(19) Europäisches Patentamt
European Patent Office
Office européen des brevets

(11) **EP 3 554 199 A1**

(12) **EUROPEAN PATENT APPLICATION**

(43) Date of publication:
**16.10.2019 Bulletin 2019/42**

(51) Int Cl.:
***H05H 9/00*** *(2006.01)*     ***H05H 7/00*** *(2006.01)*

(21) Application number: **18167210.6**

(22) Date of filing: **13.04.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **ADAM S.A.**
  **1217 Meyrin (CH)**
• **Università degli studi di Bergamo**
  **24129 Bergamo (IT)**

(72) Inventors:
• **CALDARA, Michele**
  **CH-1202 Geneve (CH)**

• **GALIZZI, Francesco**
  **I-24016 San Pellegrino Terme (IT)**
• **JEFF, Adam**
  **F-01210 Ferney Voltaire (FR)**
• **CALDARA**
  **1217 Meyrin (CH)**
• **JEFF**
  **1217 Meyrin (CH)**
• **GALIZZI**
  **24129 Bergamo (IT)**

(74) Representative: **Vanzini, Christian et al**
  **Jacobacci & Partners S.p.A.**
  **Corso Emilia 8**
  **10152 Torino (IT)**

(54) **BEAM ENERGY MEASUREMENT SYSTEM**

(57)     A time-of-flight (TOF) measurement system for measuring energy of a pulsed hadron beam, wherein each pulse of the beam is structured into a series of bunches (B) of charged particles, said bunches being repeated according to a repetition rate of the order of magnitude of radiofrequency. The system comprises a first detector (1), a second detector (2) and a third detector (3) arranged along a beam path (10), each of the detectors being configured to detect the passage of a bunch (B) of charged particles and provide an output signal ($v_{PP,1}$, $v_{PP,2}$, $v_{PP,3}$) dependent on phase of the detected bunch (B), wherein the second detector (2) is spaced apart from the first detector (1) by a first distance ($L_{12}$) and wherein the third detector (3) is spaced apart from the second detector (2) by a second distance ($L_{23}$), wherein the first distance is set out in such a way as that time of flight ($t_{12}$) of the bunch (B) from the first detector (1) to the second detector (2) is approximately equal to, or lower than a repetition period ($T_{RFQ}$) of the bunches (B), and wherein the second distance is set out in such a way as that time of flight ($T_{23}$) of the bunch (B) from the second detector (2) to the third detector (3) is greater than a multiple of the repetition period ($T_{RFQ}$) of the bunches (B), and a processing unit (7) configured to
a) calculate phase shifts ($\Delta_{\varphi12}$, $\Delta_{\varphi13}$, $\Delta_{\varphi23}$) between the output signals ($v_{PP,1}$, $v_{PP,2}$, $v_{PP,3}$) of the detectors (1, 2, 3), and
b) calculate energy (E) of the pulse based on the calculated phase shifts.

$$N = \text{number of samples in } v_{PP}$$
$$G = \text{FFT}\left[v_{PP}\right]$$
$$b = \arg\max_i |G(i)| \text{ between } f_{min} \text{ and } f_{max}$$
$$\beta = b + \frac{N}{\pi} \cdot \text{atan}\left[\frac{\sin\left(\frac{\pi}{N}\right)}{\cos\left(\frac{\pi}{N}\right)+\frac{|G_b|}{|G_{b+1}|}}\right]$$
$$f_{PP} = \frac{\beta}{N} \cdot f_{sampling}$$

FIG. 6

EP 3 554 199 A1

**Description**

Background of the Invention

The present invention relates generally to beam energy measurement systems for particle accelerators.

Linear accelerators are used in radiotherapy to accelerate particles, typically electrons, protons or heavier ions including helium or carbon ions, up to energies sufficient to allow them to travel to a depth in tissue to irradiate and impart energy to a tumor. In the case of electrons they may alternatively be directed onto a target of material of large atomic number to create high energy X-rays which themselves are then used to treat a tumor at depth.

Typically hadronic and ion particles are generated in a source (for example an Electron Cyclotron Resonance Ion Source (ECRIS) or ion plasma source for protons) and injected into a linear accelerator complex where they are accelerated by high frequency radiofrequency (RF) fields up to a required energy or energies. Acceleration typically proceeds in stages, which may include a pre-accelerator stage, for example a radiofrequency quadrupole (RFQ). The production of a high energy output beam, suitable for radiotherapy treatment or other use, in practice may involve several accelerator sub-units, possibly as many as 10-14, each comprising a sequence of individual accelerator cavities connected to waveguides arranged to couple in the driving RF fields. Typical accelerator stages include drift tube linacs (DTLs), side coupled drift tube linacs (SCDTLs) and coupled cavity linacs (CCLs). The RF fields are typically produced by klystrons or magnetrons.

Typically between the source and pre-accelerator stage is a low energy beam transfer line (LEBT). Typically a medium energy beam transfer line (MEBT) is situated between each accelerator sub-unit or between groups of sub-units. The beam path from beginning to end of the accelerator complex may be many meters long and is typically shielded throughout its length.

In medical particle accelerators the beam which is produced at the output of the last accelerating sub-unit is transported to the patient through a high energy beam transfer line (HEBT). At the end of the HEBT is a nozzle which is typically arranged to direct, or scan the beam, at the target in the patient, and which also includes a dose delivery system arranged to monitor the dose delivered to the patient.

Beam profile, beam current and beam energy are typically monitored for beam diagnostics or clinical purposes and a beam energy measurement system is typically mandatory under medical device regulations. The HEBT typically has an acceptance in beam energy much higher than the treatment requirements and this could result in a beam with slightly different, or even higher, energy from that which was requested being transported to the patient.

The Dose Delivery System, installed just before the patient, has no means of measuring beam energy of the pulses delivered to the patient. Compliance with medical device regulations may require that the system should incorporate energy measurement for every beam pulse delivered to the patient.

A classical method to measure beam energy is a spectrometer-based system, which includes the use of a bending dipole in the HEBT, in combination with beam position and/or profile detectors upstream and downstream. In case the HEBT is straight, this method cannot be used.

In view of the above, there is a need for a fast response beam energy measurement system. Furthermore, there is a need for a beam energy measurement system which is not affected by the layout of the beam transfer lines.

Summary of the Invention

Accordingly, there is provided a time-of-flight (TOF) measurement system for measuring energy of a pulsed hadron beam, wherein each pulse of the beam is structured into a series of bunches of charged particles, said bunches being repeated according to a repetition rate of the order of magnitude of radiofrequency, said system comprising a first detector, a second detector and a third detector arranged along a beam path, each of said detectors being configured to detect the passage of a bunch of charged particles and provide an output signal dependent on phase of the detected bunch, wherein the second detector is spaced apart from the first detector by a first distance and wherein the third detector is spaced apart from the second detector by a second distance, wherein said first distance is set out in such a way as that time of flight of the bunch from the first detector to the second detector is equal to, or lower than a repetition period of the bunches, and wherein said second distance is set out in such a way as that time of flight of the bunch from the second detector to the third detector is greater than a multiple of the repetition period of the bunches, and processing means configured to

  a) calculate phase shifts between the output signals of the detectors, and
  b) calculate energy of the pulse based on the calculated phase shifts.

According to an embodiment, there is provided a time-of-flight (TOF) measurement system for measuring energy of a pulsed hadron beam, wherein each pulse of the beam is structured into a series of bunches of charged particles, said bunches being repeated according to a repetition rate of the order of magnitude of radiofrequency, said system comprising

a first detector, a second detector and a third detector arranged along a beam path, each of said detectors being configured to detect the passage of a bunch of charged particles and provide an output signal dependent on phase of the detected bunch, wherein the second detector is spaced apart from the first detector by a first distance and wherein the third detector is spaced apart from the second detector by a second distance, wherein said first distance is set out in such a way that the difference between the time of flight from the first detector to the second detector for bunches of the highest and lowest energies accepted by the HEBT is equal to or lower than the repetition period of the bunches, and wherein said second distance is set out in such a way as that time of flight of the bunch from the second detector to the third detector is greater than a multiple of the repetition period of the bunches, and

processing means configured to

a) calculate phase shifts between the output signals of the detectors, and
b) calculate energy of the pulse based on the calculated phase shifts.

The bunches can potentially have a very high repetition rate (up to 3 GHz). The signal strength typically depends on beam intensity as well as beam energy and can vary within a very broad range (for example, more than 3 orders of magnitude). As an example it may vary from -60 dBm to 7 dBm. The system can measure a beam pulse average energy within a range from a minimum energy of, for example, 5 MeV to a maximum energy of, for example, 230 MeV. The system is not interceptive and can be used with any kind of hadron.

Advantageously, the beam energy measurement system according to the invention may be used in a control system of a particle accelerator for radiotherapy, allowing a pulse by pulse control or monitoring of the beam energy; this means that if the energy of a pulse varies from a requested energy by a certain extent (for example by, say, 0.17 %) then the next pulse can be prevented or stopped, or mitigated in some way so that it is not delivered to the patient.

More in general, the system of the invention allows a much higher bunches repetition rate than conventional systems. Current systems do not reach 400 MHz; this system can potentially operate up to 1 GHz or higher.

Furthermore, the system allows a very high measurement repetition rate (up to 200 Hz). Moreover, it has an energy detection accuracy, which may in some embodiments be as high as 0.03 %, which makes it usable in the Beam Delivery System.

The invention is independent of the layout of the transfer lines, and since it does not comprise a spectrometer it can be used in both straight and curved transfer lines, and can detect fast energy changes in both.

Brief Description of the Drawings

Some preferred, but non-limiting, embodiments of the invention will now be described, with reference to the attached drawings, in which:

- Figure 1 shows a diagram of a detector arrangement of a beam energy measurement system according to the invention,
- Figures 2 shows a graph reporting limits on the distance and phase-shift errors for achieving 0.03% precision in the energy measurement at E = 230 MeV,

- Figure 3 shows a hardware design of a prototype system according to the invention, and

- Figures 4 to 9 show flow diagrams of an exemplary beam energy measurement method.

Detailed Description

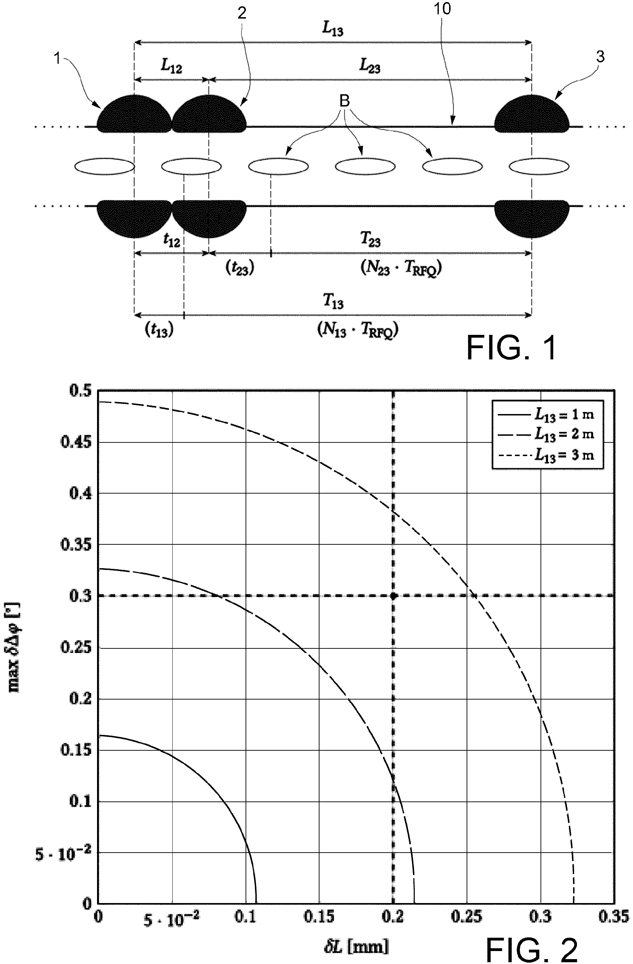

With reference to Figure 1, the energy measurement system according to the invention comprises a first detector 1, a second detector 2 and a third detector 3 arranged along a beam path 10, for example along a beam pipe of an accelerator HEBT. Each of the detectors 1, 2, 3 is configured to detect the passage of a bunch of charged particles and provide an output signal dependent on phase of the detected bunch. Beam bunches are designated with B in Figure 1. The second detector 2 is spaced apart from the first detector 1 by a first distance $L_{12}$. The third detector 3 is spaced apart from the second detector 2 by a second distance $L_{23}$. Distance between the first detector 1 and the third detector 3 is designated with $L_{13}$.

In an embodiment the detectors 1-3 are capacitive pickups and in a specific embodiment these are phase probes. In place of phase probes a beam position monitor, a beam current transformer or a wall current monitor might be used. Alternatively a resonant cavity or an electro-optic crystal may be used. In general any device which measures the electric or magnetic fields of the particle beam is suitable. In an alternative embodiment a beam loss monitor or a device that intercepts part of the beam halo could be used.

Phase probes are capacitive sensors that can be used to detect in a non-interceptive way the passage of a bunch of charged particles. Their main component is a metallic ring, placed around the beam or beam pipe, on which a charge develops when a beam bunch passes inside it. This charge can be collected to get a current proportional to the variation of charge inside the ring.

$t_{12}$ is the time taken by a particle bunch B to travel the distance $L_{12}$, which can be used to compute the particles energy:

$$\beta = \frac{L_{12}}{t_{12}} \cdot \frac{1}{c}$$

$$\gamma = \frac{1}{\sqrt{1 - \beta^2}}$$

$$E = E_0 \cdot (\gamma - 1)$$

where E is the kinetic energy of the particle and $E_0$ is the rest energy of the particle (for protons: $E_0$=938.272 MeV); c is the speed of light.

The system is designed to measure the phase shift $\Delta\phi$ between the output signal of the probes 1-3. To be able to compute $t_{12}$ from $\Delta\phi_{12}$ (i.e. the phase shift between the output signal of the first detector 1 and the output signal of the second detector 2) the relation between the two has to be unambiguous. To this end, the first distance $L_{12}$ is set out in such a way as that time of flight $t_{12}$ of the bunch B from the first detector 1 to the second detector 2 is equal to, or lower than a repetition period $T_{RFQ}$ of the bunches B. This poses a limit on the maximum value of $L_{12}$. For an energy range from 5 MeV to 230 MeV this limit is around 48 mm.

Given the limit on $L_{12}$, it is impossible to achieve 0.03 % of relative error on the measurement of E with only two probes because this would require a precision in the phase shift measurement which is nowadays unachievable. This is the reason behind the use of a third probe. $L_{23}$ is much greater than $L_{12}$, so more than one bunch can be positioned along $L_{23}$. In other words, time of flight $T_{23}$ of the bunch from the second detector 2 to the third detector 3 is greater than a multiple of the repetition period $T_{RFQ}$ of the bunches. $N_{13}$ and $N_{23}$ represent the number of whole bunches present between, respectively, detectors 1 and 3, 2 and 3. In an embodiment, the repetition rate $T_{RFQ}$ is given by the RFQ of the linear accelerator. Note that using only two distant probes is not sufficient as this would not allow an unambiguous energy measurement over the range from 5 MeV to 230 MeV.

This layout greatly improves the energy measurement precision; given the precision in the distance measurement $\delta L$ and in the phase shift measurement $\delta\Delta\phi$, the relative error on the energy measurement using only detectors 1 and 2 is

$$\frac{\beta^2 \gamma^3}{\gamma - 1} \cdot \sqrt{\left(\frac{\delta L}{L_{12}}\right)^2 + \left(\frac{\delta\Delta\varphi}{2\pi} \cdot \frac{T_{RFQ}}{t_{12}}\right)^2}$$

While when using also detector 3 it is

$$\frac{\beta^2 \gamma^3}{\gamma - 1} \cdot \sqrt{\left(\frac{\delta L}{L_{13}}\right)^2 + \left(\frac{\delta\Delta\varphi}{2\pi} \cdot \frac{T_{RFQ}}{T_{13}}\right)^2}$$

which can be reduced by increasing $L_{13}$ ($T_{13}$ also increases consequently).

Figure 2 shows another important aspect of the previous formulas; in the design phase of the accelerator layout the distance $L_{13}$ should be chosen in such way that the measurement errors on both the phase and the distance still allow to achieve the beam energy resolution needed by a medical accelerator. $\delta\Delta\phi$ is given by the electronics and from eventual interferences from high power sources in the accelerator, while $\delta L$ is the result of the accuracy of survey instruments commonly used in the accelerator alignment, like a laser tracker (typical accuracy better than $\pm50\mu$m) combined with the uncertainty on the ring electrode alignment with respect to external references (typical accuracy $\pm50\mu$m). Figure 2 depicts the upper limits on $\delta L$ and $\delta\Delta\varphi$ for achieving 0.03 % precision in the measurement of a beam at 230MeV at different values for $L_{13}$. The allowed combinations of $\delta\Delta\phi$ and $\delta L$ are those sitting below the curves, and the dot corresponding to the actual system performance is indicated.

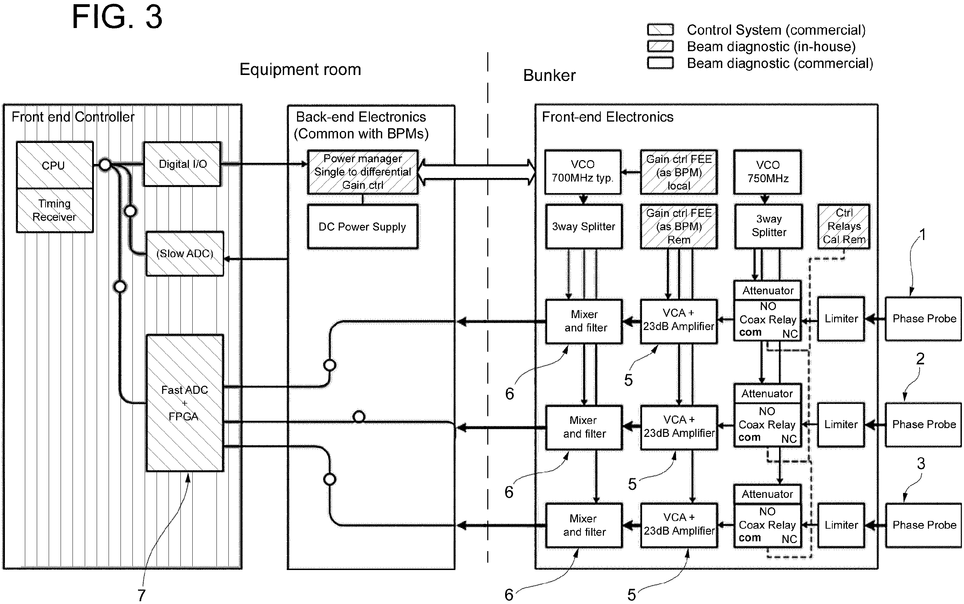

An example of hardware design which allows to measure the phase shifts between the output signals of the phase probes is shown in Figure 3. The signals formed on the detectors 1, 2, 3 at beam passage enter limiters, devices used only to protect downstream electronics from unwanted spikes on the signals and successively they pass through RF coaxial relays, where they are routed to preamplification stages (at 5), where it is eventually possible to remotely change the gain. The amplified signals are then mixed down from 750 MHz to about 50 MHz (at 6), before being transmitted along coaxial cables that exit the machine room and enters directly a fast ADC plus FPGA (at 7) for acquisition and processing. The down-mix is not necessary, it is an implementation choice to relax requirements on the ADC and processing. The coaxial relays above mentioned are used to inject at the beginning of the three electronic chains a calibration signal at the same frequency of the bunches repetition rate ($1/T_{RFQ}$), so to acquire and subtract in the data processing the fixed contribution in phase offset between the three channels due to mismatches in the cable lengths and in the installed electronics blocks (amplifiers, mixers, etc). The calibration of the system should run only once for a few milliseconds and it can be repeated any time during 'no beam' operation (for example before a new treatment starts) so to eventually update the phase offset; in this way it is possible to compensate any kind of long-term effect on the system like temperature drifts in the accelerator room.

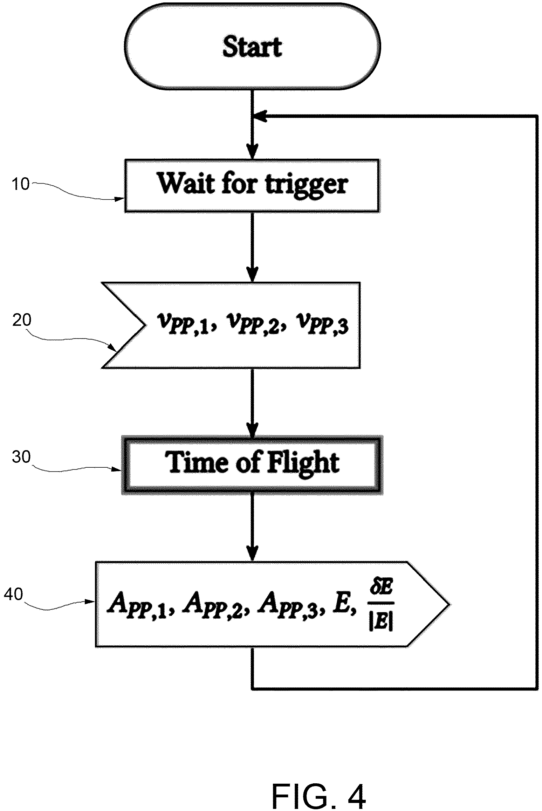

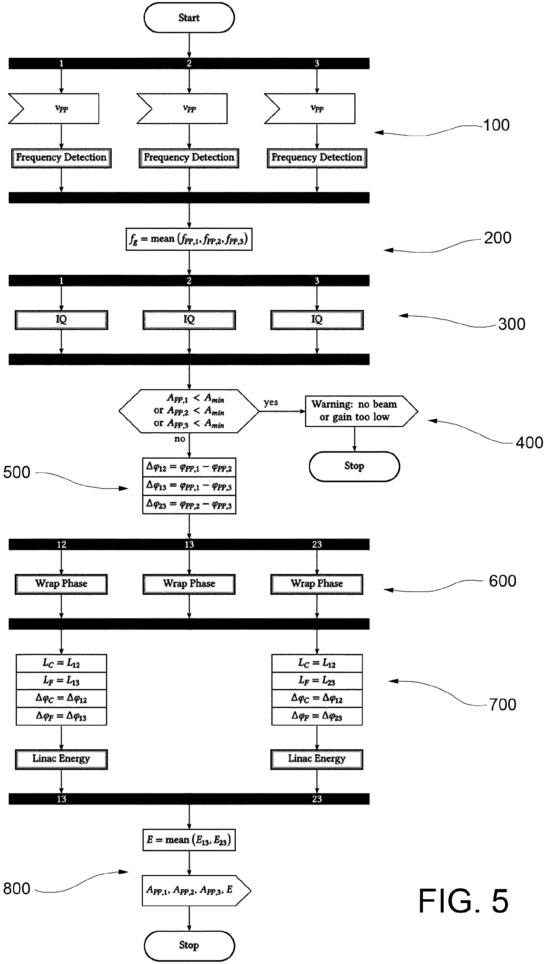

The diagrams in figures 4-9 represent an example of the computation that takes place inside the FPGA 7, which extracts the energy information from the detectors signals. The algorithm can be resumed in the following steps (Figures 4 and 5):

- Acquire the signals $v_{PP,1}$, $v_{PP,2}$, $v_{PP,3}$ coming from the detectors 1-3 (step 20). The exact time at which the acquisition should start (step 10) is computed based on a system-level trigger signal (which tells when the next pulse will occur). In a further embodiment it may also be based on the requirements that the detector signals are acquired for a duration which is slightly shorter than the pulse duration and starting slightly after the pulse so that the central part of the actual pulse is sampled.

- Perform a time-of-flight analysis of the signals (step 30). As will be explained in the following, this analysis comprises:

  - Detect the exact frequency fg of the signals $v_{PP,1}$, $v_{PP,2}$, $v_{PP,3}$. This is necessary because the down-mixing introduces some uncertainty in the signal frequency.

  - Use the detected frequency fg to perform an I/Q method on the signals, which gives the amplitude $A_{PP,1}$, $A_{PP,2}$, $A_{PP,3}$ and the phase $\varphi_{PP,1}$, $\varphi_{PP,2}$, $\varphi_{PP,3}$ of each signal.

  - Check the signals amplitudes $A_{PP,1}$, $A_{PP,2}$, $A_{PP,3}$ to see if the pulse has been correctly detected: If not, stop.

  - Use the signals phases $\varphi_{PP,1}$, $\varphi_{PP,2}$, $\varphi_{PP,3}$ to compute the energy E, and broadcast this information across the control system (step 40).

In figures 4-9, the following blocks are used:

Start/Stop: Where the (sub)algorithm flow starts or stops.

Input/Read/Acquire: In the main diagram, this means that a signal is acquired by the hardware. In sub-diagrams, this means that a variable that has previously been set by the caller is required for this sub-algorithm to work.

(continued)

Output/Write/Send: In the main diagram, this means that some kind of value is transmitted by the algorithm to some other processing unit that may be interested in that value. In sub-diagrams, this means that a variable that has been set by the sub-algorithm is made available to the caller.
Computation: Perform some kind of computation.

Decision/Branching: A point in the diagram where the algorithm flow can take different paths based on a predicate.

Sub-diagram: Execute the specified sub-diagram.

Every variable should be set before it is used or alternatively it is set as an a priori known value; the latter is true for constants known from physics and for the following variables: $A_{min}$: Minimum amplitude required for the beam pulse to be considered correctly detected. $f_{sampling}$: Sampling frequency.

A further explanation is required for the correct interpretation of the flow diagrams: Different flows enclosed by black horizontal lines represents operations that can be performed in parallel.

A label put at the top of a parallel branch has to be considered as additional subscript to every variable that appears in that branch, including input and output variables in sub-diagrams. If the label is at the bottom, only output variables gains the subscript.

With reference to Figure 5, the frequencies $f_{PP,1}$, $f_{PP,2}$, $f_{PP,3}$ of the output signals of the detectors 1-3 are detected (at 100).

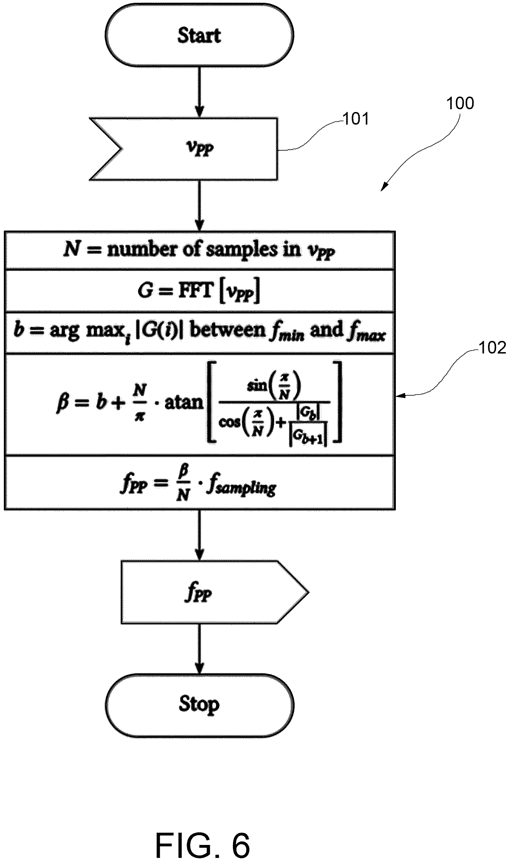

Figure 6 show this detection procedure in detail. The output signal $v_{PP}$ of each detector is sampled with the sampling frequency $f_{sampling}$ (at 101). Then, the following variables are calculated at 102:

$N$ = number of samples in $v_{PP}$,
$G$ = Fast Fourier Transform of $v_{PP}$,
$b$ = arg $\max_i |G(i)|$ between $f_{min}$ and $f_{max}$, wherein $f_{min}$ and $f_{max}$ are minimum and maximum values which can be set to constrain the search for the maximum in the Fast Fourier Transform of $v_{PP}$. They can be used when unknown frequencies are present in the Transform, although this should not be the normal situation.

$$\beta = b + \frac{N}{\pi} \cdot \mathrm{atan}\left[\frac{\sin\left(\frac{\pi}{N}\right)}{\cos\left(\frac{\pi}{N}\right)+\frac{|G_b|}{|G_{b+1}|}}\right] \cdot$$

Then, the frequency $f_{PP}$ of each signal is calculated as

$$f_{PP} = \frac{\beta}{N} \cdot f_{sampling} \cdot$$

The frequency fg of the signal is then calculated (at 200 in Figure 5) as

$$f_g = \mathrm{mean}(f_{PP,1}, f_{PP,2}, f_{PP,3}).$$

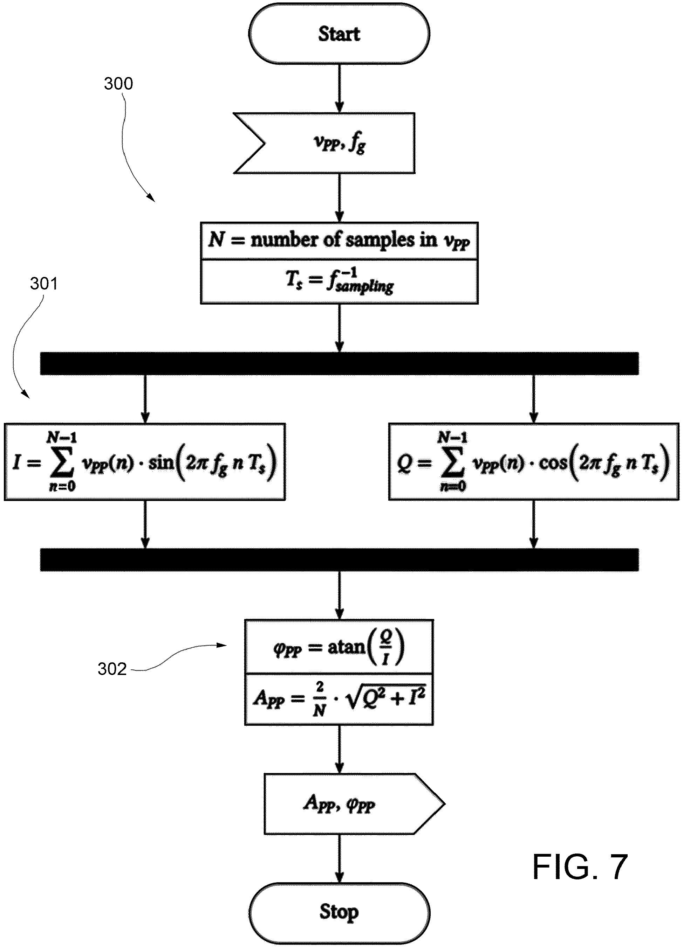

Then, an I/Q method is performed on each of the output signals $v_{PP}$ (at 300). The I/Q method is shown in detail in Figure 7. The I and Q signals are calculated at 301 as

$$I = \sum_{n=0}^{N-1} v_{PP}(n) \cdot \sin\left(2\pi f_g n T_s\right),$$

$$Q = \sum_{n=0}^{N-1} v_{PP}(n) \cdot \cos\left(2\pi f_g n T_s\right).$$

where $T_s = f^{-1}{}_{sampling}$ is the sampling period.

Then, phase $\varphi_{PP}$ and amplitude $A_{PP}$ of each signal are calculated at 302 as

$$\varphi_{PP} = \text{atan}\left(\frac{Q}{I}\right),$$

$$A_{PP} = \frac{2}{N} \cdot \sqrt{Q^2 + I^2}.$$

The signal amplitudes $A_{PP}$ are then compared with $A_{min}$ (at 400 in Figure 5). If at least one of these amplitudes is lower than $A_{min}$, the process is stopped.

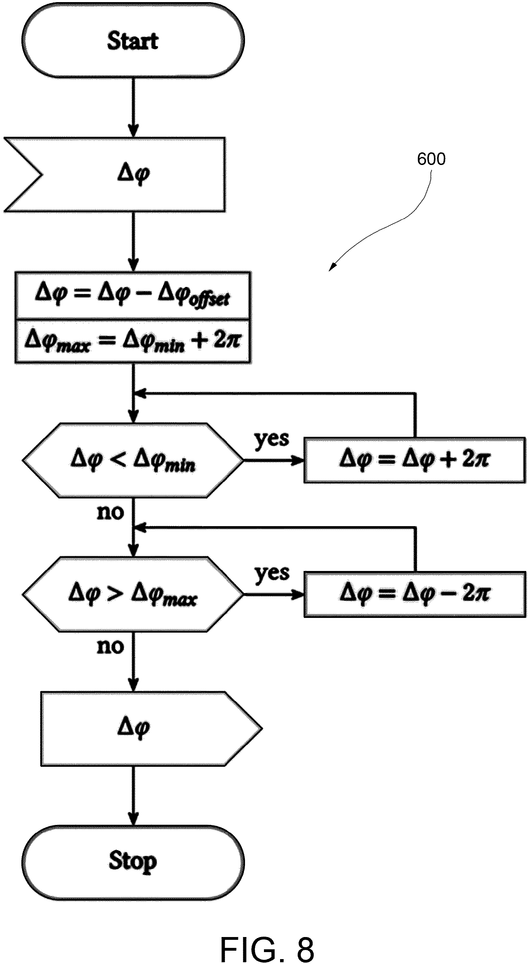

Otherwise, the phase shifts $\Delta\varphi$ of the output signals are calculated at 500, and subjected to wrapping at 600 (see also Figure 8).

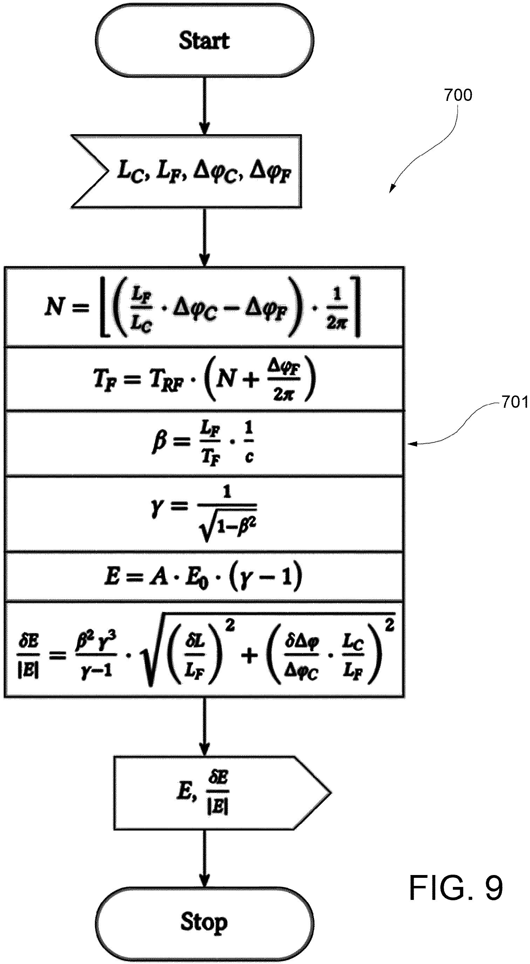

Then, energy values $E_{13}$ and $E_{23}$ of the pulse is calculated (at 700) based on time-of-flight measurements between detectors 1 and 3, and between detectors 2 and 3, respectively. This calculation is shown in detail in Figure 9. The following variables are calculated at 701 (here, the subscript C takes place of subscript 12, while subscript F takes place of subscript 13 or 23, depending on whether $E_{13}$ or $E_{23}$ is calculated; $T_{RF}$ corresponds to $T_{RFQ}$ mentioned above):

$$N = \left\lfloor \left(\frac{L_F}{L_C} \cdot \Delta\varphi_C - \Delta\varphi_F\right) \cdot \frac{1}{2\pi} \right\rceil,$$

$$T_F = T_{RF} \cdot \left(N + \frac{\Delta\varphi_F}{2\pi}\right),$$

$$\beta = \frac{L_F}{T_F} \cdot \frac{1}{c},$$

$$\gamma = \frac{1}{\sqrt{1-\beta^2}},$$

$$E_{(13 \, or \, 23)} = A \cdot E_0 \cdot (\gamma - 1),$$

$$\frac{\delta E}{|E|} = \frac{\beta^2\gamma^3}{\gamma - 1} \cdot \sqrt{\left(\frac{\delta L}{L_F}\right)^2 + \left(\frac{\delta\Delta\varphi}{\Delta\varphi_C} \cdot \frac{L_C}{L_F}\right)^2}$$

The energy E of the pulse is calculated as a mean value between $E_{13}$ and $E_{23}$ (at 800).

According to an alternative embodiment, it would be sufficient to use $E_{13}$ or $E_{23}$ to provide the beam energy. The mean value between $E_{13}$ and $E_{23}$ is used to improve the measurement accuracy. The accuracy might be even further improved using a fourth detector/phase probe or more, but this would add complexity to the system.

The ToF beam energy measurement system allows a high accuracy beam energy measurement to be made at a very high measurement rate (up to 200 Hz) and provides the result typically within 1 ms from the passage of the beam pulse, making it suitable to be used not only as Beam Diagnostics device but also in the Beam Delivery System to monitor each beam pulse average energy, which has been delivered to the patient.

Such a highly responsive system is fast enough to allow the system to take actions to disable generation of the next beam pulse.

The system according to the invention makes no assumptions about the speed at which the beam energy can be changed, thus it poses no restrictions on the energy change rate. This is an improvement over the state-of-the-art because current beam energy measurement systems are either destructive or they do not allow the measurement of fast beam energy changes.

**Claims**

1. A time-of-flight (TOF) measurement system for measuring energy of a pulsed hadron beam, wherein each pulse of the beam is structured into a series of bunches (B) of charged particles, said bunches being repeated according to a repetition rate of the order of magnitude of radiofrequency, said system comprising
   a first detector (1), a second detector (2) and a third detector (3) arranged along a beam path (10), each of said detectors being configured to detect the passage of a bunch (B) of charged particles and provide an output signal ($v_{PP,1}$, $v_{PP,2}$, $v_{PP,3}$) dependent on phase of the detected bunch (B), wherein the second detector (2) is spaced apart from the first detector (1) by a first distance ($L_{12}$) and wherein the third detector (3) is spaced apart from the second detector (2) by a second distance ($L_{23}$), wherein said first distance is set out in such a way as that time of flight ($t_{12}$) of the bunch (B) from the first detector (1) to the second detector (2) is approximately equal to, or lower than a repetition period ($T_{RFQ}$) of the bunches (B), and wherein said second distance is set out in such a way as that time of flight ($T_{23}$) of the bunch (B) from the second detector (2) to the third detector (3) is greater than a multiple of the repetition period ($T_{RFQ}$) of the bunches (B), and
   processing means (7) configured to

   a) calculate phase shifts ($\Delta\varphi_{12}$, $\Delta\varphi_{13}$, $\Delta\varphi_{23}$) between the output signals ($v_{PP,1}$, $v_{PP,2}$, $v_{PP,3}$) of the detectors (1, 2, 3), and
   b) calculate energy (E) of the pulse based on the calculated phase shifts.

2. A system according to claim 1, wherein said step a) comprises
   detecting a frequency ($f_g$) of the output signals ($v_{PP,1}$, $v_{PP,2}$, $v_{PP,3}$) of the detectors (1, 2, 3), and
   performing an I/Q method on the output signals ($v_{PP,1}$, $v_{PP,2}$, $v_{PP,3}$), based on the detected frequency ($f_g$), to calculate the amplitude ($A_{PP,1}$, $A_{PP,2}$, $A_{PP,3}$) and phase ($\varphi_{PP,1}$, $\varphi_{PP,2}$, $\varphi_{PP,3}$) of each output signal ($v_{PP,1}$, $v_{PP,2}$, $v_{PP,3}$).

3. A system according to claim 1 or 2, wherein said detectors are detectors responsive to an electric field or magnetic field of the pulsed hadron beam passing thereby.

4. A system according to claim 1 or 2, wherein said detectors are detectors intercepting a fraction of the pulsed hadron beam.

5. A system according to any of claims 1 to 4, wherein the repetition rate of the bunches (B) is of an order of magnitude comprised between 100 MHz and 3 GHz, and preferably comprised between 100 MHz and 1 GHz.

6. Radiotherapy apparatus comprising at least one linear accelerator configured to produce and accelerate a hadron beam, and further comprising a beam energy measurement system according to any of the preceding claims.

7. Apparatus according to claim 6, wherein said linear accelerator is configured to produce and accelerate a proton beam.

FIG. 1

FIG. 2

# FIG. 3

Legend:
- Control System (commercial)
- Beam diagnostic (in-house)
- Beam diagnostic (commercial)

**Bunker**

Phase Probe 1
Phase Probe 2
Phase Probe 3

Front-end Electronics

Ctrl Relays Cal Rem

Limiter

VCO 750MHz — 3way Splitter

Attenuator — NO Coax Relay com NC

Gain ctrl FEE (as BPM) local
Gain ctrl FEE (as BPM) Rem

VCA + 23dB Amplifier — 5

VCO 700MHz typ. — 3way Splitter

Mixer and filter — 6

**Equipment room**

Back-end Electronics (Common with BPMs)

Power manager Single to differential Gain ctrl

DC Power Supply

Front end Controller

Digital I/O

(Slow ADC)

Fast ADC + FPGA — 7

CPU
Timing Receiver

10

FIG. 4

Start

| 1 | 2 | 3 |

$v_{PP}$ | $v_{PP}$ | $v_{PP}$

100

Frequency Detection | Frequency Detection | Frequency Detection

$$f_g = \text{mean}\left(f_{PP,1}, f_{PP,2}, f_{PP,3}\right)$$

200

| 1 | 2 | 3 |

IQ | IQ | IQ

300

$A_{PP,1} < A_{min}$
or $A_{PP,2} < A_{min}$
or $A_{PP,3} < A_{min}$

yes → Warning: no beam or gain too low

400

no

Stop

500

$$\Delta\varphi_{12} = \varphi_{PP,1} - \varphi_{PP,2}$$
$$\Delta\varphi_{13} = \varphi_{PP,1} - \varphi_{PP,3}$$
$$\Delta\varphi_{23} = \varphi_{PP,2} - \varphi_{PP,3}$$

| 12 | 13 | 23 |

Wrap Phase | Wrap Phase | Wrap Phase

600

$L_C = L_{12}$
$L_F = L_{13}$
$\Delta\varphi_C = \Delta\varphi_{12}$
$\Delta\varphi_F = \Delta\varphi_{13}$

$L_C = L_{12}$
$L_F = L_{23}$
$\Delta\varphi_C = \Delta\varphi_{12}$
$\Delta\varphi_F = \Delta\varphi_{23}$

700

Linac Energy | Linac Energy

| 13 | 23 |

$$E = \text{mean}\left(E_{13}, E_{23}\right)$$

800

$A_{PP,1}, A_{PP,2}, A_{PP,3}, E$

Stop

FIG. 5

FIG. 6

FIG. 7

FIG. 8

Start

700

$$L_C, L_F, \Delta\varphi_C, \Delta\varphi_F$$

701

$$N = \left\lfloor \left( \frac{L_F}{L_C} \cdot \Delta\varphi_C - \Delta\varphi_F \right) \cdot \frac{1}{2\pi} \right\rfloor$$

$$T_F = T_{RF} \cdot \left( N + \frac{\Delta\varphi_F}{2\pi} \right)$$

$$\beta = \frac{L_F}{T_F} \cdot \frac{1}{c}$$

$$\gamma = \frac{1}{\sqrt{1-\beta^2}}$$

$$E = A \cdot E_0 \cdot (\gamma - 1)$$

$$\frac{\delta E}{|E|} = \frac{\beta^2 \gamma^3}{\gamma - 1} \cdot \sqrt{\left( \frac{\delta L}{L_F} \right)^2 + \left( \frac{\delta\Delta\varphi}{\Delta\varphi_C} \cdot \frac{L_C}{L_F} \right)^2}$$

$$E, \frac{\delta E}{|E|}$$

Stop

FIG. 9

Europäisches
Patentamt

European
Patent Office

Office européen
des brevets

# EUROPEAN SEARCH REPORT

Application Number

EP 18 16 7210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | M.MARCHETTO ET AL.: "Beam Quality and Operational Experience with the Superconducting Linac at the ISAC II RIB Facility", PROCEEDINGS OF PAC07, ALBUQUERQUE, NEW MEXICO, USA, 1 August 2007 (2007-08-01), pages 1392-1394, XP002786246, | 1,2,4-7 | INV. H05H9/00 H05H7/00 |
| Y | * page 1393, column 1 - page 1394, column 1 * | 3 | |
| | ----- | | |
| X | R.LAXDAL: "Commissioning and Early Experiments with ISAC-II", PROCEEDINGS OF PAC07, ALBUQUERQUE, NEW MEXICO, USA, 1 August 2007 (2007-08-01), pages 2593-2597, XP002786247, * page 2595, column 2 * | 1,2,4-7 | |
| | ----- | | |
| X | V.A.VERZILOV ET AL.: "Time Domain Diagnostics for the ISAC-II Superconducting Heavy Ion Linac", PROCEEDINGS OF DIPAC2007, VENICE, ITALY, 31 October 2008 (2008-10-31), pages 247-249, XP002786262, * page 247, column 2 - page 248, column 2 * | 1,2,4-7 | TECHNICAL FIELDS SEARCHED (IPC) H05H |
| | ----- | | |
| Y | JI-GWANG HWANG ET AL: "High Precision Capacitive Beam Phase Probe for KHIMA Project", NUCLEAR INSTRUMENTS AND METHODS IN PHYSICS RESEARCH A 837 (2016), 24 August 2016 (2016-08-24), pages 34-39, XP002785963, | 3 | |
| A | * abstract * * paragraph [0002] * | 5 | |
| | ----- -/-- | | |

The present search report has been drawn up for all claims

| Place of search | Date of completion of the search | Examiner |
|---|---|---|
| The Hague | 6 November 2018 | Crescenti, Massimo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 16 7210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAN-SUNG KIM: "Characterisation of Output Beam from KOMAC Accelerator", JOURNAL OF THE KOREAN PHYSICAL SOCIETY, vol. 71, no. 11, 1 December 2017 (2017-12-01), pages 807-813, XP036436110, * page 807, column 2 * ----- | 1-7 | |
| A | R.C.PARDO ET AL.: "Energy Measurement Using a Resonator Based Time-of-Flight System", IEEE TRANSACTIONS ON NUCLEAR SCIENCE, vol. 30, no. 4, 4 August 1983 (1983-08-04), pages 2237-2238, XP002785954, * Introduction; figure 1 * ----- | 1-7 | |

|  | TECHNICAL FIELDS SEARCHED (IPC) |
|---|---|

The present search report has been drawn up for all claims

| Place of search | Date of completion of the search | Examiner |
|---|---|---|
| The Hague | 6 November 2018 | Crescenti, Massimo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)